(19) Europäisches Patentamt
European Patent Office
Office européen des brevets

(11) Veröffentlichungsnummer: **0 000 724**
B1

(12) **EUROPÄISCHE PATENTSCHRIFT**

(45) Veröffentlichungstag der Patentschrift: **08.04.81**

(51) Int. Cl.³: **C 08 G 18/77,** C 08 G 18/71, C 07 C 143/68, C 08 G 18/50, C 08 G 18/46

(21) Anmeldenummer: **78100487.4**

(22) Anmeldetag: **24.07.78**

(54) **Hydroxylgruppen und Urethano-aryl-sulfonsäuregruppen enthaltende Verbindungen und Verfahren zu deren Herstellung; Verwendung dieser Verbindungen als Reaktionskomponente für Polyisocyanate.**

(30) Priorität: **03.08.77 DE 2735013**

(43) Veröffentlichungstag der Anmeldung: **21.02.79 Patentblatt 79/4**

(45) Bekanntmachung des Hinweises auf die Patenterteilung: **08.04.81 Patentblatt 81/14**

(84) Benannte Vertragsstaaten: **BE DE FR GB**

(56) Entgegenhaltungen:
**FR-A-1 546 668**
**FR-A-2 264 010**

(73) Patentinhaber: **BAYER AG, Zentralbereich Patente, Marken und Lizenzen, D-5090 Leverkusen 1, Bayerwerk (DE)**

(72) Erfinder: **Dieterich, Dieter, Dr., Ludwig-Girtler-Strasse 1, D-5090 Leverkusen (DE)**
Erfinder: **Ballé, Gerhard, Dr., Nietzschestrasse 14, D-5090 Leverkusen (DE)**
Erfinder: **Schmelzer, Hans Georg, Dr., Weissdornweg 13, D-5024 Pulheim (DE)**

**EP 0 000 724 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchgebühr entrichtet worden ist (Art. 99(1) Europäisches Patentübereinkommen).

# Hydroxylgruppen und Urethano-aryl-sulfonsäuregruppen enthaltende Verbindungen und Verfahren zu deren Herstellung; Verwendung dieser Verbindungen als Reaktionskomponente für Polyisocyanate

Es ist bekannt, zur Herstellung von Polyurethanen Verbindungen zu verwenden, welche zwei bis sechs OH-Gruppen enthalten und ein Molekulargewicht zwischen 62 und etwa 10 000 aufweisen. Zu diesen Polyhydroxyverbindungen zählen beispielsweise:
einfache, höherfunktionelle Alkohole, wie z. B. Äthylenglykol, Diäthylenglykol, Hexandiol, Glycerin, Trimethylolpropan sowie höhermolekulare Polyäther, Polythioäther, Polyester, Polyacetale. Diese höhermolekularen Polyhydroxyverbindungen werden in bekannter Weise aus niedermolekularen Bausteinen hergestellt. Im allgemeinen sind diese Hydroxyverbindungen wenig polar und tragen keine weiteren funktionellen Gruppen.

Bei der Herstellung von Schaumstoffen aus Polyisocyanaten und Polyhydroxyverbindungen sind im allgemeinen oberflächenaktive Verbindungen, insbesondere aus der Gruppe der Organo-Polysiloxane, erforderlich. Diese Produkte bewirken eine Emulgierung der Reaktionskomponenten und vor allem eine Stabilisierung des zunächst noch flüssigen Schaumgerüsts. Auch bei der Herstellung nicht-zelliger Polyurethane werden zuweilen Emulgatoren mitverwendet, wenn mangelnde Verträglichkeit der Reaktionskomponenten oder die Mitverwendung von Füllstoffen dies erforderlich machen. In vielen Fällen muß der Emulgator bzw. Stabilisator als separate Komponente dem Reaktionsgemisch zugesetzt werden, was wegen der im allgemeinen geringen Mengen dieser Komponenten Dosierprobleme mit sich bringen kann.

Es wäre ein Vorteil, wenn beispielsweise bei Unverträglichkeit der Reaktanten auf spezielle oberflächenaktive Verbindungen verzichtet werden könnte, indem schon die verwendeten Polyole selbst die gewünschten oberflächenaktiven Eigenschaften aufwiesen. Es besteht also ein Bedarf für Polyole mit oberflächenaktiven Eigenschaften. Ferner besteht der Wunsch nach Polyolen höherer Polarität und Hydrophilie, um auf diese Weise die Wasserverträglichkeit von Polyolen zu verbessern und den aus den Polyolen hergestellten Schaumstoffen ein gewisses Wasseraufnahmevermögen sowie eine verbesserte Lösungsmittelresistenz zu verleihen. Ferner besteht ein Bedarf nach Polyhydroxylverbindungen, welche Polyurethane mit verbessertem Brandverhalten liefern. Schließlich wären OH-Präpolymere wünschenswert, welche beim hydrolytischen Abbau keine toxikologisch bedenklichen aromatischen Diamine liefern.

Die vorliegende Erfindung liefert eine Lösung dieser Probleme. Überraschenderweise wurde nämlich gefunden, daß durch Umsetzung von Polyhydroxyverbindungen mit molar unterschüssigen Mengen aromatischer Isocyanatosulfonsäuren, gegebenenfalls im Gemisch mit üblichen Polyisocyanaten, neuartige Hydroxyverbindungen erhalten werden, welche erhöhte Polarität sowie oberflächenaktive Eigenschaften aufweisen und z. B. daraus hergestellten Schaumstoffen verbessertes Brandverhalten sowie Hochfrequenzverschweißbarkeit verleihen.

Gegenstand der vorliegenden Erfindung sind somit mindestens eine OH-funktionelle Kette und mindestens eine Urethano-arylsulfonsäuregruppe aufweisende Verbindungen vom durchschnittlichen Molekulargewicht 300 bis 12 000.

Erfindungsgemäß bevorzugt sind dabei Verbindungen, welche ein durchschnittliches Molekulargewicht von 400 bis 12 000 aufweisen, gekennzeichnet durch mindestens eine OH-funktionelle Langkette, welche 15 bis 400 Kettenglieder, vorzugsweise 30 bis 300 Kettenglieder, enthält. Kettenglieder sind z. B. $CH(CH_3)$- oder $CH_2$-Gruppen, Äthersauerstoffatome, CO-Gruppen, Schwefelatome und/oder Stickstoffatome.

Die erfindungsgemäßen Verbindungen enthalten bevorzugt mindestens eine Strukturelement der allgemeinen Formel:

$$[-(O)_{1-5}-R_1-O-CO-NH]_{1-3}-Ar-[SO_2-OH]_{1-2}$$

in der

$R_1$ einen 2- bis 6wertigen Rest eines Polyols, z. B. eines Polyesters, Polyäthers, Polythioäthers oder Polyesteramids und

Ar einen mehrwertigen Rest eines aromatischen Isocyanats darstellt.

Erfindungsgemäß sind Verbindungen folgender allgemeiner Formeln bevorzugt:

$$[(HO)_{1-5}-R_1-O-CO-NH]_2-Ar-[SO_2-OH]_{1-2}$$

Gegenstand der vorliegenden Erfindung ist auch ein Verfahren zur Herstellung von mindestens eine OH-funktionelle Kette und mindestens eine Urethano-aryl-sulfonsäuregruppe aufweisenden Verbindungen vom durchschnittlichen Molekulargewicht 300 bis 12 000, dadurch gekennzeichnet, daß Verbindungen vom Molekulargewicht 62 bis 10 000, die mindestens zwei Hydroxylgruppen aufweisen, bei 0–190°C mit aromatischen Isocyanatosulfonsäuren umgesetzt werden, wobei das Äquivalent-Verhältnis der Gesamtmenge an Isocyanatgruppen (einschließlich der gegebenenfalls in dimerisierter

Form vorhandenen Isocyanatgruppen) zu Sulfonsäuregruppen 0,5 bis 50 und das Äquivalent-Verhältnis aus der Summe der Hydroxylgruppen der mindestens zwei Hydroxylgruppen aufweisenden Verbindungen zu NCO-Gruppen 1,5 bis 30 beträgt.

Gegenstand der vorliegenden Erfindung ist schließlich auch die Verwendung der erfindungsgemäßen Verbindungen als Reaktionskomponente für Polyisocyanate zur Herstellung von Polyadditionsprodukten oder Polykondensationsprodukten.

Die neuen erfindungsgemäßen Verbindungen besitzen gegenüber den bisher bekannten Polyhydroxyverbindungen eine Reihe von vorteilhaften Eigenschaften:

1. Sie besitzen stark polaren bzw. oberflächenaktiven Charakter, außerordentlich niedrigen Dampfdruck und sind hervorragend verträglich mit einer Vielzahl polarer und apolarer Medien und Reaktionspartnern.
2. In Abhängigkeit von Art und Menge der eingesetzten Isocyanatoarylsulfonsäure sowie Art und Menge der zur Neutralisation der Sulfonsäure-Gruppen verwendeten Basen lassen sich die oberflächenaktiven Eigenschaften sowie die Hydrophilie in weiten Grenzen wunschgemäß steuern.
3. Der hydrolytische Abbau der Produkte führt zu toxikologisch unbedenklichen Aminosulfonsäuren.
4. Die Verwendung der erfindungsgemäßen Verbindungen beispielsweise bei der Herstellung von Polyurethanen führt zu Polymeren mit verbessertem Brandverhalten.

Bei der Durchführung des erfindungsgemäßen Verfahrens erfolgt eine Addition eines Teils der OH-Gruppen der als Ausgangsmaterial verwendeten Polyhydroxyverbindungen mit den NCO-Gruppen sowie gegebenenfalls vorhandenen Uretdion-Gruppen der Isocyanatoarylsulfonsäure unter Ausbildung von höhermolekularen neuen Polyhydroxyverbindungen, welche zumindest anteilig Urethan-Gruppen und eine oder mehrere freie Sulfonsäure-Gruppen enthalten. Die Sulfonsäure-Gruppen können anschließend mit üblichen anorganischen oder organischen Basen ganz oder teilweise neutralisiert werden.

Beim erfindungsgemäßen Verfahren können als Ausgangsmaterial alle in der Polyurethanchemie üblicherweise verwendeten, mindestens zwei Hydroxylgruppen aufweisenden Verbindungen vom Molekulargewicht 62 – 10 000 eingesetzt werden. So eignen sich beispielsweise:
niedermolekulare Glykole, Polyester, Polyäther, Polyesteramide, OH-funktionelle Oligomere, Polymerisate, beispielsweise auf Basis Butadien sowie durch Vinylmonomere gepfropfte Polyäther oder auch solche Polyäther, welche andere Polymere, wie z. B. Polyharnstoffe, Harnstoffharze, Polyhydrazodicarbonamide oder Vinylpolymerisate dispergiert enthalten. Beispiele für geeignete hydroxyfunktionelle Verbindungen sind im folgenden aufgeführt.

Die in Frage kommenden Hydroxylgruppen aufweisenden Polyester sind z. B. solche, wie sie in der DE-A 2 647 482, Seiten 8 – 9, genannt werden.

Auch die erfindungsgemäß in Frage kommenden, zwei Hydroxylgruppen aufweisenden Polyäther, durch Vinylpolymerisate modifizierten Polyäther, Polythioäther, Polyacetale, Hydroxylgruppen aufweisende Polycarbonate und Polyesteramide und Polyamide sind z. B. solche, wie sie in der DE-A 2 647 482, Seiten 9, 10 und 11, beschrieben werden.

Erfindungsgemäß können jedoch auch Polyhydroxylverbindungen eingesetzt werden, in welchen hochmolekulare Polyaddukte bzw. Polykondensate in feindisperser oder gelöster Form enthalten sind. Derartige modifizierte Polyhydroxylverbindungen werden erhalten, wenn man Polyadditionsreaktionen (z. B. Umsetzungen zwischen Polyisocyanaten und aminofunktionellen Verbindungen) bzw. Polykondensationsreaktionen (z. B. zwischen Formaldehyd und Phenolen und/oder Aminen) direkt in situ in den oben genannten, Hydroxylgruppen aufweisenden Verbindungen ablaufen läßt. Derartige Verfahren sind beispielsweise in den Deutschen Auslegeschriften 1 168 075 und 1 260 142 sowie den Deutschen Offenlegungsschriften 2 324 134, 2 423 984, 2 512 385, 2 513 815, 2 550 796, 2 550 797, 2 550 833 und 2 550 662 beschrieben. Es ist aber auch möglich, gemäß US-Patent 3 869 413 bzw. Deutscher Offenlegungsschrift 2 550 860 eine fertige wäßrige Polymerdispersion mit einer Polyhydroxylverbindung zu vermischen und anschließend aus dem Gemisch das Wasser zu entfernen.

Niedermolekulare Glykole, welche im Gemisch mit den genannten höhermolekularen Polyhydroxylverbindungen oder auch allein mit Isocyanatosulfonsäuren umgesetzt werden können, sind z. B.: Äthylenglykol, Diäthylenglykol, Triäthylenglykol, Tetraäthylenglykol, Propylenglykol, Oligopropylenglykol, Butandiol, Hexandiol, 2-Äthylhexandiol, Octandiol, Glycerin, Trimethylpropan, Dodecandiol. Auch Aminoalkohole wie Äthanolamin, Propanolamin, Diäthanolamin können eingesetzt werden unter der Voraussetzung, daß alle vorhandenen Aminogruppen mit Isocyanatgruppen zur Reaktion gebracht werden. In untergeordneten Mengen können auch Mono-, Di- oder Polyamine sowie Wasser Verwendung finden. Die nach der Umsetzung erhaltenen Produkte sollen außer OH-Gruppen höchstens in untergeordneten Mengen Carboxylgruppen oder Mercaptogruppen enthalten.

Beim erfindungsgemäßen Verfahren können als Isocyanatoarylsulfonsäuren die Sulfonierungsprodukte aller bekannten aromatischen Polyisocyanate eingesetzt werden. Beispiele für derartige in Form ihrer Sulfonierungsprodukte beim erfindungsgemäßen Verfahren einsetzbare aromatische Polyisocyanate sind solche, wie sie in der DE-A 2 647 482, Seiten 5 und 6, beschrieben werden.

Geeignet sind auch Phosgenierungsprodukte von Kondensaten von Anilin und Aldehyden oder Ketonen, wie z. B. Acetaldehyd, Propionaldehyd, Butyraldehyd, Aceton, Methyläthylketon. Ferner geeignet sind die Phosgenierungsprodukte von Kondensaten von am Kern Alkyl-substituierten Anilinen, insbesondere Toluidinen, mit Aldehyden oder Ketonen, wie z. B. Formaldehyd, Acetaldehyd, Butyraldehyd, Aceton, Methyläthylketon.

Weiterhin geeignet sind Umsetzungsprodukte der genannten aromatischen Polyisocyanatgemische mit 0,2—50 Mol-% an Polyolen, vorausgesetzt, daß die Viskosität der so erhaltenen Umsetzungsprodukte 50 000 cP bei 25°C nicht überschreitet und der NCO-Gehalt der Umsetzungsprodukte mindestens 6 Gew.-% beträgt. Geeignete Polyole zur Modifizierung der Ausgangsmaterialien sind insbesondere die in der Polyurethan-Chemie bekannten Polyäther- und/oder Polyesterpolyole des Molekulargewichtsbereichs 200 bis 6000, vorzugsweise 300 bis 4000, sowie niedermolekulare Polyole des Molekulargewichtsbereichs 62 bis 200. Beispiele derartiger niedermolekularer Polyole sind Äthylenglykol, Propylenglykol, Glyzerin, Trimethylolpropan, 1,4,6-Hexantriol.

Besonders bevorzugte Isocyanatoaryl-sulfonsäuren sind die Sulfonierungsprodukte von 2,4-Toluylendiisocyanat sowie Gemischen aus 2,4- und 2,5-Toluylendiisocyanat, ferner Sulfonierungsprodukte der Di- bzw. Polyisocyanate, welche durch Phosgenierung von Anilin/Formaldehyd-Kondensaten erhalten teilweiser Sulfonierung aromatischer Polyisocyanate erhalten. Im allgemeinen erhält man bei der Teilsulfonierung von chemisch einheitlichen Diisocyanaten oder von binären Isomerengemischen Suspensionen, während bei der Teilsulfonierung von Mehrkomponenten-Gemischen homogene Lösungen entstehen. Für das erfindungsgemäße Verfahren ist es grundsätzlich ohne Belang, ob Lösungen oder Suspensionen eingesetzt werden. Ganz besonders bevorzugt sind teilsulfonierte Polyisocyanatgemische, wie sie durch Phosgenierung von Anilin-Formaldehyd-Kondensaten erhalten werden und in den deutschen Offenlegungsschriften 2 227 111, 2 359 614 und 2 359 615 beschrieben sind. Ebenfalls besonders bevorzugt sind Suspensionen von Diisocyanato-toluol-sulfonsäure-Dimeren sowie Diisocyanatodiphenylmethan-sulfonsäure-Dimeren in Diisocyanatotoluol bzw. Diisocyanatodiphenylmethan.

Die Herstellung der beim erfindungsgemäßen Verfahren einzusetzenden Isocyanatoarylsulfonsäuren bzw. ihrer Gemische mit nicht sulfonierten aromatischen Polyisocyanaten erfolgt nach den bekannten Verfahren des Standes der Technik bzw. in Analogie zu den bekannten Verfahren des Standes der Technik, wie er sich beispielsweise aus den bereits genannten Veröffentlichungen, oder aus US-A 3 826 769 ergibt. Die Verfahren der DE-A 2 524 476 oder 2 615 876 sind zur Herstellung von beim erfindungsgemäßen Verfahren einsetzbaren Isocyanatoarylsulfonsäuren ebenfalls geeignet.

Es ist auch möglich, beim erfindungsgemäßen Verfahren Lösungen bzw. Suspensionen der beispielhaft genannten Isocyanatoarylsulfonsäuren in aliphatischen Polyisocyanaten wie z. B. Tetramethylendiisocyanat oder Hexamethylendiisocyanat und/oder in cycloaliphatischen bzw. gemischt aliphatisch-cyclo-aliphatischen Polyisocyanaten wie z. B. 4,4'-Diisocyanato-dicyclohexylmethan, 2,4- bzw. 2,6-Diisocyanato-hexahydrotoluol oder 1-Isocyanato-3,3,5-trimethyl-5-isocyanatomethylcyclohexan einzusetzen. Falls eine Erniedrigung der NCO-Funktionalität der erfindungsgemäßen Verfahrensprodukte erwünscht ist können auch Lösungen bzw. Suspensionen der Isocyanato-arylsulfonsäuren in aromatischen, aliphatischen oder cycloaliphatischen Monoisocyanaten zum Einsatz gelangen. Beispiele für die letztgenannten Verbindungen sind Phenylisocyanat, Tosylisocyanat, n-Hexylisocyanat, 6-Chlor-hexylisocyanat, Cyclohexylisocyanat oder Methoxymethylisocyanat. Grundsätzlich denkbar ist auch der Einsatz von sulfonierten aromatischen Monoisocyanaten, wie z. B. Phenylisocyanat als Isocyanatoarylsulfonsäure in Kombination mit nicht sulfonierten Polyisocyanaten der beispielhaft genannten Art. Die Art und Mengenverhältnisse der beim erfindungsgemäßen Verfahren einzusetzenden Isocyanate, sowie der Sulfonierungsgrad werden häufig so gewählt, daß das Äquivalentverhältnis von gegebenenfalls teilweise in dimerisierter Form vorliegenden Isocyanatgruppen zu Sulfonsäuregruppen $>1:1$ d. h insbesondere zwischen 1,05 : 1 und 50 : 1, vorzugsweise zwischen 2 : 1 und 30 : 1, liegt. Ganz besonders bevorzugt ist ein Verhältnis zwischen 2 : 1 und 12 : 1.

Eine weitere Gruppe von bevorzugten Isocyanatosulfonsäuren sind solche aromatischen Mono-, Di- oder Polyisocyanate, welche mehr als eine Sulfonsäuregruppe und insbesondere zwei oder drei Sulfonsäuregruppen enthalten. Solche Isocyanatopolysulfonsäuren sind in der DE-A 2 615 876 beschrieben. Sofern Monoisocyanatodisulfonsäuren (mit) eingesetzt werden, kann das Äquivalentverhältnis von NCO-Gruppen zu $SO_3H$-Gruppen auch zwischen 1 : 1 und 0,5 : 1 betragen.

Zur Herstellung von Hydroxyverbindungen mit endständigen Sulfonsäure- bzw. Sulfonatgruppen werden vorzugsweise Monoisocyanatosulfonsäuren eingesetzt, z. B. die Sulfonierungsprodukte von Phenylisocyanat, m-Tolylisocyanat, p-Tolylisocyanat, p-Chlorphenylisocyanat, p-Nitrophenylisocyanat, p-Methoxyphenylisocyanat, p-Chlormethyl-phenylisocyanat, m-Chlorphenylisocyanat, m-Chlormethyl-phenylisocyanat.

Das Mengenverhältnis zwischen Polyhydroxyverbindungen und Isocyanatosulfonsäure wird meist so gewählt, daß OH-funktionelle Produkte eines Molekulargewichts unter 12 000 und vorzugsweise unter 6000 entstehen. Es wird also ein molarer Überschuß an hydroxyfunktionellen Komponenten eingesetzt, wobei auf eine NCO-Gruppe mindestens 1,5 OH-Gruppen entfallen sollen. Unter NCO-Gruppen sollen dabei nicht nur in freier Form vorliegende NCO-Gruppen verstanden werden,

sondern auch in Form von Uretdion-Gruppen vorliegende dimerisierte NCO-Gruppen. Es ist besonders bevorzugt, die als Ausgangsmaterial verwendeten hydroxyfunktionellen Verbindungen nur anteilig mit Sulfonsäure-Gruppen zu modifizieren, wobei auf eine NCO-Gruppe bis zu 30 OH-Gruppen eingesetzt werden können. Bevorzugt ist ein Äquivalent-Verhältnis von OH-Gruppen zu NCO-Gruppen zwischen 2 und 20.

Die genannten Mengenverhältnisse gelten im wesentlichen für Umsetzungen, die unter Verwendung von Di- bzw. Polyisocyanaten durchgeführt werden und die unmittelbar zu durch Sulfonsäuregruppen modifizierten Polyhydroxyverbindungen führen.

Im Rahmen der vorliegenden Erfindung können jedoch auch Monoisocyanate, welche 1 bis 3 Sulfonsäuregruppen enthalten, Verwendung finden. Diese Monoisocyanate werden mit den Ausgangs-Hydroxyverbindungen in molar unterschüssigen Mengen umgesetzt.

Bevorzugt ist ein Äquivalentverhältnis von OH-Gruppen zu NCO-Gruppen zwischen 2 und 6.

Die Umsetzung der Ausgangs-Hydroxyverbindungen mit den Sulfonsäuregruppen enthaltenden Isocyanaten erfolgt im Prinzip in bekannter Weise. Im allgemeinen werden die Hydroxyverbindungen vorgelegt und Isocyanatkomponente unter Vermischen zugegeben. Ist das Isocyanat flüssig, wie es beispielsweise bei teilsulfonierten MDI-Typen der Fall ist, so kann die Vermischung der Komponenten und die anschließende Reaktion ohne weiteres bei Raumtemperatur oder auch bei geringfügig erhöhter Temperatur stattfinden. Die Wahl der Temperatur hängt in diesem Fall ausschließlich von der Viskosität des Reaktionsgemisches und von der gewünschten Zeitdauer der Umsetzung ab. Bei Verwendung von festen Isocyanatoaryl-, Mono- oder Polysulfonsäuren entsteht bei der Vermischung primär eine Suspension und es ist zweckmäßig, die Umsetzung bei einer Temperatur vorzunehmen, bei der das feste Isocyanat rasch in Lösung geht.

Hierfür sind Temperaturen zwischen 40 und 180°C, insbesondere 60 und 120°C zweckmäßig. Insbesondere bei ausschließlichem Einsatz verhältnismäßig niedermolekularer Polyhydroxyverbindungen werden Temperaturen über 120°C bis etwa 200°C bevorzugt, um ein Festwerden des Reaktionsansatzes während der Umsetzung zu vermeiden. Feste Isocyanatosulfonsäuren werden besonders bevorzugt in Form von Suspensionen, Pasten oder Feuchtpulvern, unter Verwendung inerter Lösungsmittel eingesetzt, wie dies in der DE-A 2 640 103 beschrieben ist.

Weiterhin können feste Isocyanatosulfonsäuren in der Form von Lösungen in organischen Lösungsmitteln eingesetzt werden, wobei als Lösungsmittel flüssige Ester einer anorganischen oder organischen Säure des Phosphors bevorzugt werden (DE-A 2 650 172).

Im übrigen können selbstverständlich beliebige inerte Lösungsmittel, wie Kohlenwasserstoffe, Halogenkohlenwasserstoffe, Äther, Ester und Ketone dem Reaktionsgemisch zugegeben werden. Bevorzugt ist indessen die Umsetzung in Abwesenheit von Lösungsmitteln bzw. mit den geringen Lösungsmittelmengen welche zum Anpasten oder Lösen fester Isocyanatosulfonsäuren Verwendung finden.

Eine bevorzugte Arbeitsweise besteht in der Herstellung unsymmetrischer Hydroxyverbindungen unter Ausnutzung der unterschiedlichen Reaktionsfähigkeit der Isocyanatgruppen. So kann man beispielsweise eine Diisocyanatoarylsulfonsäure zunächst mit einem monofunktionellen Alkohol, einer Fettsäure, einem Aminoalkohol, prim. oder sek. Aminumsetzen, z. B. zu 20—70% und anschließend die verbleibenden NCO-Gruppen mit einer Di- bzw. Polyhydroxyverbindung zur Reaktion bringen. In Abhängigkeit von Art und Menge der monofunktionellen wie auch der polyfunktionellen Komponente können die oberflächenaktiven Eigenschaften vielfältig variiert werden.

Die zusätzlich zu den bereits aufgeführten Polyhydroxyverbindungen einsetzbaren monofunktionellen Verbindungen umfassen z. B. Methanol, Äthanol, Isopropanol, n-Butanol, Glykolmonomethyläther, Glykolmonoäthyläther, Diglykolmonomethyläther, n-Octanol, n-Dodecanol, Oleylalkohol, Stearylalkohol, hydroxyfunktionelle Fettsäureester des Glycerins, Trimethylolpropans, und Trimethyloläthans, Stearinsäure, Kokosfettsäure, Leinölfettsäure, Sojafettsäure, Aminoäthanol, Aminopropanol (derartige Aminoalkohole können im Rahmen der oben beschriebenen Arbeitsweise infolge der stark unterschiedlichen Reaktivität von Amino- und Hydroxy-Funktion näherungsweise als monofunktionell angesehen werden), Butylamin, sec. Butylamin, Kokosfettamin.

Die stufenweise Herstellung derartiger unsymmetrischer Hydroxyverbindungen wird besonders bevorzugt in einem Lösungsmittel durchgeführt, z. B. in Aceton oder einem organischen Phosphorsäureester. Dabei werden kurzkettige hydrophile monofunktionelle Verbindungen vorzugsweise mit überwiegend hydrophoben Polyhydroxyverbindungen und langkettige hydrophobe monofunktionelle Verbindungen vorzugsweise mit hydrophilen Polyhydroxyverbindungen kombiniert.

Die Sulfonsäuregruppen enthaltenden Hydroxyverbindungen können mit anorganischen oder organischen Basen ganz oder teilweise neutralisiert werden. Geeignete Neutralisationsmittel sind z. B. organische Basen wie monofunktionelle primäre, sekundäre und tertiäre Amine wie beispielsweise Methylamin, Diäthylamin, Triäthylamin, Trimethylamin, Dimethylamin, Äthylamin, Tributylamin, Pyridin, Anilin, Toluidin, alkoxylierte Amine wie Äthanolamin, Diäthanolamin, Triäthanolamin, Methyldiäthanolamin, Dimethylaminoäthanol, Oleyldiäthanolamin, sowie polyfunktionelle Polyamine, bei denen die einzelnen Aminogruppen gegebenenfalls unterschiedliche Basizität aufweisen können, wie z. B. die durch Hydrierung von Additionsprodukten von Acrylnitril an primäre und sekundäre Amine erhaltenen Polyamine, per- oder partiell alkylierte Polyamine wie N,N-Dimethyläthylendiamin, ferner

Verbindungen wie α-Aminopyridin, N,N-Dimethylhydrazin; anorganische Basen, basisch reagierende oder basenabspaltende Verbindungen wie Ammoniak, einwertige Metallhydroxide, -carbonate und -oxide wie Natriumhydroxid, Kaliumhydroxid.

Ferner eignen sich als Neutralisationsmittel Guanidine, Guanidincarbonat, Harnstoff, Methylharnstoff, Dimethylharnstoff, Caprolactam, Dimethylformamid, Dimethylacetamid, Pyrrolidon, sowie feste anorganische Basen wie Calciumoxid, Calciumhydroxid, Calciumcarbonat, Magnesiumoxid, Magnesiumcarbonat, Dolomit, Lithiumhydroxid, Lithiumcarbonat, Zinkoxid, Zinkcarbonat sowie basische anorganische Füllstoffe.

Schwach basische Neutralisationsmittel wie Harnstoff oder Caprolactam, sowie basische Füllstoffe können ohne weiteres auch in Überschuß gegenüber den vorhandenen Sulfonsäuregruppen angewandt werden.

Die erfindungsgemäßen Produkte sind wertvolle Ausgangsmaterialien zur Herstellung von Polyurethankunststoffen nach dem Isocyanat-Polyadditionsverfahren. Sie eignen sich z. B. zur Herstellung von kompakten oder zelligen Elastomeren, Weichschäumen und Hartschäumen, insbesondere dann, wenn hohe Anforderungen an die Vernetzungsdichte, das Brandverhalten oder die Abbaubarkeit gestellt werden. So eignen sich die erfindungsgemäßen Polyhydroxyverbindungen beispielsweise zur Herstellung von Polstermaterialien, Matratzen, elastischen Unterlagen, Autositzen, Dampfungsmaterialien, Stoßabsorbern, Konstruktionswerkstoffen, schalldämmenden Isolierungen, feuchtigkeitsaufnehmenden Materialien, z. B. im Hygienesektor, zur Herstellung von Substraten zur Pflanzenaufzucht, sowie für den Wärme- und Kälteschutz. Ganz besonders geeignet sind die erfindungsgemäßen Polyhydroxyverbindungen zur Herstellung anorganisch-organischer Kunststoffe beispielsweise in Analogie zu den in DE-C 2 310 559, DE-A 2 227 147, 2 359 608 beschriebenen Verfahrensweisen, sowie für Oberflächenbeschichtungen, Imprägnierungen und Verklebungen geeignet.

Ferner eignen sich die Produkte gemäß vorliegender Erfindung als vielseitig zu verwendende oberflächenaktive Verbindungen, z. B. als Emulgatoren. Schaumstabilisatoren, als Färbereihilfsmittel, als Flotationsmittel und zur Herstellung von Polyurethandispersionen.

Ein besonderer Vorteil der erfindungsgemäßen Hydroxy-Verbindungen ist ihre erhöhte Polarität. Dadurch sind diese Produkte im Gegensatz beispielsweise zu reinen Polypropylenglykoläthern mit niedermolekularen Glykolen wie Äthylenglykol, Diäthylenglykol, 1,4-Butandiol, Glycerin, gut verträglich. Mischungen sind homogen und damit lagerstabil.

Zur Herstellung von Polyadditionsprodukten mit günstigem Brandverhalten ist die Umsetzung der Polyhydroxyverbindungen gemäß vorliegender Erfindung mit Sulfonsäureester-Gruppen aufweisenden Polyisocyanaten besonders günstig.

Der Einsatz der erfindungsgemäßen Verbindungen empfiehlt sich insbesondere dann, wenn Polyisocyanat-Komponenten und Polyhydroxi-Komponenten infolge Unverträglichkeit zunächst Emulsionen bilden, die erst nach einer gewissen Induktionszeit homogen werden. Bereits sehr geringe Mengen der erfindungsgemäßen Produkte begünstigen die Bildung besonders feinteiliger Emulsionen, die wesentlich rascher reagieren. Weiterhin beeinflussen die neuen Produkte die Porenstruktur von daraus hergestellten Schaumstoffen und bewirken in vielen Fällen eine wünschenswerte Erhöhung der Stauchhärte. Schließlich eignen sich die Produkte auch zur Hydrophobierung oberflächenmodifizierter anorganischer Füllstoffe.

## Beispiel 1

2000 g eines auf ein Gemisch aus 84% Trimethylolpropan und 16% 1,2-Propylenglykol gestarteten Polypropylenglykoläthers der OH-Zahl 42 werden mit 62 g toluolfeuchtem Uretdion der Diisocyanatotoluolsulfonsäure (hergestellt aus Toluylendiisocyanat, Isomerengemisch 2,4 : 2,6 = 80 : 20), entsprechend 40 g Trockensubstanz bei Raumtemperatur innig vermischt. Innerhalb von 45 Minuten wird die Suspension auf 100° C geheizt und 3 Stunden bei 100 – 125° C gehalten. Toluol wird bei 100° C und 1866 Pa (14 Torr) abgezogen und die Lösung filtriert.

Schwefel-Gehalt: 0,24%
Viskosität: 2200 mPa · s
pH-Wert einer Lösung von 1 g Produkt in 90 g Methanol und 10 g Wasser: 2,7

## Beispiel 2

1021 g des Produkts gemäß Beispiel 1 werden mit 38 g 15prozentiger methanolischer Kalilauge versetzt und bei 35° C das Methanol im Vakuum abgezogen.

pH-Wert: 8
Viskosität: 1700 mPa · s

Beispiel 3

Es wird wie in Beispiel 1 verfahren, jedoch unter Verwendung von 154 g (100 g Trockensubstanz) an Uretdion.

Viskosität: 32 000 mPa · s

Beispiel 4

1064 g des Produkts gemäß Beispiel 3 werden mit 83 g 15prozentiger methanolischer Kalilauge versetzt und bei 35° C das Methanol im Vakuum abgezogen.

pH-Wert: 8
Viskosität: 90 000 mPa · s

Beispiel 5

4000 g eines auf Trimethylolpropan gestarteten Polypropylenglykoläthers mit 13% endständigen Äthylenglykoläthergruppen der OH-Zahl 35 werden mit 123 g (80 g Trockensubstanz) des im Beispiel 1 beschriebenen Uretdions bei Raumtemperatur innig vermischt. Es wird unter Stickstoff 1 Stunde bei 25—27° C gerührt, dann auf 50° C erwärmt und 4 Stunden bei dieser Temperatur gehalten, währenddessen durch Anlegen von Wasserstrahlvakuum das Toluol abgezogen wurde. Dabei ging der größte Teil des Uretdions in Lösung. Anschließend wird noch 8 Stunden bei 60—65° C untergerührt und die Lösung filtriert. Auf dem Filter verbleiben ca. 0,3 g Rückstand.

OH-Zahl: 36,4;
Säure-Zahl: 4,7

Beispiel 6

2060 g des Produkts gemäß Beispiel 5 werden mit 77 g 15prozentiger methanolischer Kalilauge versetzt und bei 35° C das Methanol im Vakuum abgezogen.

pH-Wert: 8
OH-Zahl: 32,6
Säurezahl: 0,2

Beispiel 7

Es wird wie in Beispiel 5 verfahren, jedoch ausgehend von einem entsprechenden Polyäther der OH-Zahl 28. Es wird ein gelblich-brauner viskoser modifizierter Polyäther erhalten.

OH-Zahl: 28,1;
Säure-Zahl: 4,7.

Beispiel 8

2060 g des Produkts gemäß Beispiel 7 werden mit 69 g 15%iger methanolischer Kalilauge versetzt und bei 35° C das Methanol im Vakuum abgezogen.

pH-Wert: 7,5
OH-Zahl: 21,4
Säurezahl: 0,2

Beispiel 9

200 g Uretdion der Diisocyanatotoluolsulfonsäure (vgl. Beispiel 1) werden mit 373 g Toluol verrieben und mit 10 kg eines auf Trimethylolpropan gestarteten Polypropylenglykoläthers mit 17% endständigen Äthylenglykoläthergruppen der OH-Zahl 35 bei 50° C unter Rühren vermischt.

Anschließend wird die Temperatur auf 60° C erhöht und durch Anlegen von Wasserstrahlvakuum das Toluol abgezogen. Innerhalb von 9 Stunden ging das Uretdion praktisch quantitativ in Lösung. Der modifizierte Polyäther wird zum Schluß bei 60° C über ein feines Metallsieb filtriert.

OH-Zahl: 34,5;
Säure-Zahl: 4,3;
Schwefel-Gehalt: 0,2%.

Wird das Uretdion vor der Zugabe zum Polyäther mit 200 g Tris-chlor-äthylphosphat anstelle des Toluols verrieben, so geht das Produkt schon nach kurzer Zeit bei 60° C in Lösung.

## Beispiel 10

380 g toluolfeuchtes Uretdion der Diisocyanatotoluolsulfonsäure (vgl. Beispiel 1), entsprechend 300 g Trockensubstanz, werden mit 550 g Toluol gründlich verrieben und zu 15 kg eines auf Trimethylolpropan gestarteten Polypropylenglykoläthers mit 13% endständigen Äthylenglykoläthergruppen der OH-Zahl 28 bei 50° C unter Rühren zugefügt. Anschließend wird auf 65° erwärmt und 5 Stunden gerührt, währenddessen der größte Teil des Uretdions in Lösung geht. Während weiteren 3 Stunden Rührens bei 65° C wird das Toluol im Wasserstrahlvakuum abdestilliert, das Produkt wird bei 50° C über ein Metallsieb von ca. 4 g Ungelöstem abfiltriert.

OH-Zahl: 22,2;
Säure-Zahl: 4,7;
Schwefelgehalt: 0,22%;
Toluolgehalt: 0,6%.

## Beispiel 11

Zu 5000 g des nach Beispiel 10 erhaltenen Produktes werden bei Raumtemperatur innerhalb 1 Stunde 156 g 15%ige methanolische Kalilauge unter Rühren zugetropft. Anschließend wird im Wasserstrahlvakuum bei bis zu 55° C das Methanol abdestilliert. Der erhaltene Sulfonatgruppen aufweisende Polyäther hat eine Viskosität von 3400 mPa · s.

OH-Zahl: 21,2;
Säurezahl: 0,9.

## Beispiel 12

Zu 5000 g des nach Beispiel 10 erhaltenen Polyäthers werden bei Raumtemperatur innerhalb von 2 Stunden 42 g Triäthylamin zugetropft. Anschließend wird noch 5 Stunden bei Raumtemperatur nachgerührt.

Viskosität: 3400 mPa · s;
OH-Zahl: 23,9.

## Beispiel 13

Zu 4800 g des gemäß Beispiel 10 erhaltenen Polyäthers werden 143 g bis Bis-(2-Hydroxiäthyl)-oleylamin zugetropft. Anschließend wird 4 Stunden bei Raumtemperatur nachgerührt.

Viskosität: 3800 mPa · s;
OH-Zahl: 34,4.

Für die Verschäumungsversuche wurden folgende Materialien eingesetzt:

Polyol A: Polyoxyalkylenäthertriol vom Äquivalentgewicht 2000 mit endständigen Polyoxyäthylenblöcken und einem Anteil an primären Hydroxylgruppen von über 80%.
Polyol B: Das Sulfonsäuregruppen enthaltende Polyätherpolyol aus Beispiel 8, neutralisiert mit KOH.
Polyol C: Das Sulfonsäuregruppen enthaltende Polyätherpolyol aus Beispiel 11, neutralisiert mit KOH.
Polyol D: Entspricht Polyol C, jedoch neutralisiert mit Triäthylamin (Beispiel 12).
Polyol E: Entspricht Polyol C, jedoch neutralisiert mit N,N-Dihydroxyäthyloleylamin (Beispiel 13).

Dabco: 1,4-Diazabicyclooctan (Triäthylendiamin)
TCAP: Tris-(2-chloräthyl)-phosphat

## Beispiel 14

In einem Pappbecher wurden folgende Komponenten eingewogen und 60 Sekunden lang mit einem hochtourigen Rührwerk intensiv verrührt:

| | |
|---|---|
| 100 Gew.-Teile | Polyol A |
| 100 Gew.-Teile | Polyol B |
| 6,4 Gew.-Teile | Wasser |
| 0,2 Gew.-Teile | Dabco |
| 7,2 Gew.-Teile | Diisopropanolamin |
| 2,0 Gew.-Teile | Triäthanolamin |
| 2,0 Gew.-Teile | Diäthanolamin |
| 4,0 Gew.-Teile | TCAP |
| 1,0 Gew.-Teile | eines kurzkettigen Polyphenylsiloxan-Stabilisators gemäß DE-A 2 232 525 |

In die homogene Mischung wurden 55,4 Gew.-Teile (entsprechend einem NCO/OH-Index = 100) eines modifizierten Toluylendiisocyanats (Desmodur MT 58 Allophanat-TDI) gegeben und schnell untergemischt. Nach 5 Sekunden begann das Gemisch aufzuschäumen. Es wurde sofort in eine aus Papier gefaltete quadratische Form gegossen und schäumte innerhalb von 60 Sekunden auf und war 10 Sekunden nach beendetem Aufschäumen abgebunden. Es entstand ein hochelastischer Schaumstoff mit feiner Zellstruktur, einem Raumgewicht von 36 g/l und guter Stauchhärte und Zugfestigkeit. Ein 2 cm starker und 10 cm breiter Streifen des Schaums wurde an einem Ende mit der entleuchteten Flamme eines Bunsenbrenners beflammt. Der Schaum brannte unter schwacher Rauchentwicklung und Abschmelzen, verlosch jedoch kurze Zeit nach Entfernen der Bunsenflamme.

## Beispiele 15—17

In der in Beispiel 14 beschriebenen Weise wurden nach folgenden Rezepturen Schaumstoffe hergestellt (Mengen in Gew.-Teilen):

| | 15 | 16 | 17 |
|---|---|---|---|
| Polyol C | 100 | — | — |
| Polyol D | — | 100 | — |
| Polyol E | — | — | 100 |
| Wasser | 3,2 | 3,2 | 3,2 |
| Dabco | 0,2 | 0,2 | 0,2 |
| Diisopropanolamin | 3,6 | 3,6 | 3,6 |
| Triäthanolamin | 1,0 | 1,0 | 1,0 |
| Diäthanolamin | 1,0 | 1,0 | 1,0 |
| TCAP | 2,0 | 2,0 | 2,0 |
| Polyphenylsiloxan wie in Beispiel 1 | 3,0 | 3,5 | 3,0 |
| Bis-2-N,N-Dimethylaminoäthyläther | 0,2 | 0,2 | 0,25 |
| modifiziertes Toluylendiisocyanat | 55,2 | 55,2 | 55,2 |
| NCO/OH-Index | 100 | 100 | 100 |

Bei der Verschäumung wurden folgende Reaktionszeiten beobachtet:

| | a | b | c |
|---|---|---|---|
| Rührzeit (Startzeit) | 10″ | 6″ | 6″ |
| Steigzeit | 90″ | 80″ | 90″ |
| Abbindezeit | 25″ | 20″ | 20″ |

Die erhaltenen Schaumstoffe entsprechen in mechanischen Eigenschaften und Brandverhalten dem Schaum aus Beispiel 14.

Vergleichsbeispiel

Zum Vergleich wurde nach der gleichen Rezeptur ein Schaumstoff unter alleiniger Verwendung von Polyol A hergestellt.

| | | |
|---|---|---|
| Polyol A | 100 Gew.-Teile | |
| Wasser | 3,2 Gew.-Teile | |
| Dabco | 0,1 Gew.-Teile | |
| Diisopropanolamin | 3,6 Gew.-Teile | |
| Triäthanolamin | | 1,0 Gew.-Teile |
| Diäthanolamin | | 1,0 Gew.-Teile |
| TCAP | | 2,0 Gew.-Teile |
| Polyphenylsiloxan wie in Beispiel 1 | | 1,0 Gew.-Teile |
| modifiziertes Toluylendiisocyanat | | 56,3 Gew.-Teile |
| NCO/OH-Index | | 100 |

Folgende Reaktionszeiten wurden beobachtet:

| | |
|---|---|
| Rührzeit | 15″ |
| Steigzeit | 240″ |
| Abbindezeit | 165″ |

Der Vergleichsschaum benötigt zum Aufsteigen und Aushärten erheblich längere Zeitspannen als die in den Beispielen beschriebenen Schaumstoffe. Auch bleibt die Oberfläche deutlich länger klebrig.

**Patentansprüche**

1. Mindestens eine OH-funktionelle Kette und mindestens eine Urethano-aryl-sulfonsäure-Gruppe aufweisende Verbindungen vom durchschnittlichen Molekulargewicht 300 bis 12 000.

2. Verbindungen gemäß Anspruch 1, gekennzeichnet durch mindestens eine OH-funktionelle Kette, welche 15 bis 400 Kettenglieder enthält, sowie durch ein Molekulargewicht von 400 bis 12 000.

3. Verbindungen gemäß Anspruch 2, gekennzeichnet durch mindestens eine OH-funktionelle Kette, welche 30 bis 300 Kettenglieder enthält.

4. Verfahren zur Herstellung von mindestens eine OH-funktionelle Kette und mindestens eine Urethano-aryl-sulfonsäure-Gruppe aufweisenden Verbindungen vom durchschnittlichen Molekulargewicht 300 bis 12 000, dadurch gekennzeichnet, daß Verbindungen vom Molekulargewicht 62 bis 10 000, die mindestens zwei Hydroxylgruppen aufweisen, bei 0—190°C mit aromatischen Isocyanatosulfonsäuren umgesetzt werden, wobei das Äquivalent-Verhältnis der Gesamtmenge an Isocyanatgruppen (einschließlich der gegebenenfalls in dimerisierter Form vorhandenen Isocyanatgruppen) zu Sulfonsäuregruppen 0,5 bis 50 und das Äquivalent-Verhältnis aus der Summe der Hydroxyl-Gruppen der Verbindungen mit mindestens zwei Hydroxylgruppen zu NCO-Gruppen 1,5 bis 30 beträgt.

5. Verwendung der Verbindungen gemäß Anspruch 1 als Reaktionskomponente für Polyisocyanate zur Herstellung von Polyadditionsprodukten und/oder Polykondensationsprodukten.

**Claims**

1. Compounds having an average molecular weight of from 300 to 12,000 having at least one OH-functional chain and at least one urethano-aryl-sulphonic acid group.

2. Compounds according to Claim 1, characterised by at least one OH-functional chain containing from 15 to 400 chain members and by a molecular weight of from 400 to 12,000.

3. Compounds according to Claim 2, characterized by at least one OH-functional chain which contains from 30 to 300 chain members.

4. Process for the preparation of compounds having an average molecular weight of from 300 to 12,000 having at least one OH-functional chain and at least one urethano-aryl-sulphonic acid group, characterised in that compounds having a molecular weight of from 62 to 10,000 having at least two hydroxyl groups are reacted at from 0 to 190°C with aromatic isocyanato sulphonic acids, the equivalent ratio of the total quantity of isocyanate groups (including any isocyanate groups present in dimerised form) to sulphonic acid groups being from 0.5 to 50 and the equivalent ratio of the sum of hydroxyl groups in the compounds having at least two hydroxyl groups to NCO groups being from 1.5 to 30.

5. Use of compounds according to Claim 1 as reactants for polyisocyanates for the production of polyaddition products and/or polycondensation products.

**Revendications**

1. Composés comportant au moins une chaîne à fonctionnalité OH et au moins un groupe d'acide uréthano-aryl-sulfonique, ces composés ayant un poids moléculaire moyen de 300 à 12.000.
2. Composés suivant la revendication 1, caractérisés en ce qu'ils comportent au moins une chaîne à fonctionnalité OH contenant 15 à 400 chaînons, ces composés ayant un poids moléculaire de 400 à 12.000.
3. Composés suivant la revendication 2, caractérisés en ce qu'ils comportent au moins une chaîne à fonctionnalité OH contenant 30 à 300 chaînons.
4. Procédé de préparation de composés comportant au moins une chaîne à fonctionnalité OH et au moins un groupe d'acide uréthano-aryl-sulfonique, ces composés ayant un poids moléculaire moyen de 300 à 12.000, caractérisé en ce qu'on fait réagir des composés d'un poids moléculaire de 62 à 10.000, comportant au moins deux groupes hydroxy, avec des acides isocyanato-sulfoniques aromatiques à une température de 0 à 190° C, le rapport équivalent entre la quantité totale des groupes isocyanates (y compris les groupes isocyanates éventuellement présents sous forme dimérisée) et les groupes d'acides sulfoniques étant de 0,5 à 50, tandis que le rapport équivalent entre la somme des groupes hydroxy des composés comportant au moins deux groupes hydroxy et les groupes NCO est de 1,5 à 30.
5. Utilisation des composés suivant la revendication 1 comme composants réactionnels pour les polyisocyanates en vue de préparer des produits de polyaddition et/ou des produits de polycondensation.